# EUROPÄISCHE PATENTSCHRIFT

(11) **EP 2 188 365 B1**
(45) Veröffentlichungstag und Bekanntmachung des Hinweises auf die Patenterteilung: **12.06.2013**
(21) Anmeldenummer: 08801171.3
(22) Anmeldetag: 08.08.2008
(51) Int. Cl.: C12M 1/34, G01N 33/48

(54) **ZELLKULTURMESSSYSTEM UND VERFAHREN FÜR VERGLEICHENDE UNTERSUCHUNGEN AN ZELLKULTUREN**
CELL CULTURE MEASURING SYSTEM AND METHOD FOR COMPARATIVE INVESTIGATIONS ON CELL CULTURES
SYSTÈME DE MESURE DE CULTURES CELLULAIRES ET PROCÉDÉ DE RÉALISATION D'ANALYSES COMPARATIVES SUR DES CULTURES CELLULAIRES

(30) Priorität: 10.08.2007 DE 102007038777
(43) Veröffentlichungstag der Anmeldung: 26.05.2010
(73) Patentinhaber: Fraunhofer-Gesellschaft zur Förderung der angewandten Forschung e.V., 80686 München (DE)
(72) Erfinder: SONNTAG, Frank, 09130 Chemnitz (DE); MEHRINGER, Florian, 95176 Konradsreuth (DE); SCHILLING, Niels, 09648 Mittweida (DE); JÄGER, Martin, 01326 Dresden (DE)
(74) Vertreter: Pfenning, Meinig & Partner GbR
(86) Internationale Anmeldenummer: PCT/DE2008/001348
(87) Internationale Veröffentlichungsnummer: WO 2009/021501

(56) Entgegenhaltungen:
- EP-A- 0 394 406
- WO-A-99/36766
- WO-A-03/082469
- WO-A-2005/106478
- WO-A-2006/047591
- DE-A1- 1 598 855
- DE-A1- 19 709 649
- DE-A1- 19 720 997
- DE-A1- 19 920 811

## Beschreibung

Die Erfindung betrifft ein Zellkulturmesssystem sowie ein Verfahren für vergleichende Untersuchungen an Zellkulturen. Sie kann insbesondere eingesetzt werden, wenn funktionelle Wirkungen auf lebende kultivierten Zellen, die infolge von Mitteln, die die Zellen beeinflussen können, ausgeübt und bewertet werden sollen. Dabei kann das Verhalten oder die Veränderung der Morphologie von Zellen unter dem Einfluss von in Testflüssigkeit enthaltenen Substanzen oder Testflüssigkeiten die solche Wirkungen hervorrufen, detektiert werden.

Dabei ist es üblicherweise gewünscht, dass vergleichende Untersuchungen an lebenden Zellkulturen durchgeführt werden, um entsprechende Aussagen über die Wirkungen von zellenbeeinflussenden Mitteln treffen zu können. Dementsprechend ist es erforderlich, dass die in unterschiedlicher Form beeinflussten Zellkulturen ansonsten unter gleichen Bedingungen kultiviert werden, so dass unterschiedliche auf Zellkulturen wirkende Einflüsse ausgeschlossen werden können.

So ist aus EP 0 394 406 B1 ein Verfahren und eine Vorrichtung zum Nachweis einer Wirkung eines zellbeeinflussenden Mittels auf lebende Zellen bekannt, bei der eine Mikrofließkammer, in der lebende Zellen kultiviert sind, von Lösungen oder Suspensionen, die ein zellbeeinflussendes Mittel enthalten durchströmt werden. Dabei werden aber alle in der Mikrofließkammer enthaltenen lebenden Zellen von dem zellbeeinflussenden Mittel beeinflusst, so dass für eine vergleichende Untersuchung zumindest eine zweite Mikrofließkammer vorhanden sein muss, in der eine Untersuchung ohne den Einfluss eines zellbeeinflussenden Mittels erfolgen kann. Durch den Einsatz mindestens zwei solcher voneinander getrennten Mikrofließkammern ist es aber problematisch, die gewünschten gleichen Bedingungen für die Zelluntersuchungen einhalten zu können. Außerdem besteht bei dieser bekannten technischen Lösung keine Möglichkeit, Untersuchung bezüglich durch zellbeeinflussende Mittel hervorgerufene Interaktionen an Zellen und auch die Erkennung einer Reizweiterleitung zwischen von dem zellbeeinflussenden Mittel beeinflussten Zellen auf von diesen Mitteln nicht beeinflussten Zellen zu erfassen.

Letztgenannter Aspekt ist aber auch bei der in DE 199 20 811 B4 beschriebenen Vorrichtung zur Durchführung von Untersuchungen an Zellkulturen problematisch. Bei dieser bekannten Vorrichtung soll ein trogförmiges Aufnahmebehältnis, in dem bodenseitig eine Zellkultur in einem flüssigen Kulturmedium enthalten ist, eingesetzt werden. Durch einen in das Aufnahmebehältnis eingesetzten Trennkörper, der bis zum Boden des Aufnahmebehältnisses eingeführt werden kann, kann ein Teilraum als Reaktionsraum vom übrigen Teil abgetrennt werden. In einem solchen Reaktionsraum können dann zellbeeinflussende Mittel auf die dort angeordneten Zellen wirken, wohingegen ein anderer Bereich des Aufnahmebehältnisses nicht von solchen Mitteln beeinflusst werden kann.

Beim Einführen eines solchen Trennkörpers, der in Form eines Stempels ausgebildet sein kann, kann es aber zu Undichtheiten kommen, so dass ein zellbeeinflussendes Mittel aus dem Reaktionsraum austreten und in den anderen Teil des Aufnahmebehälters gelangen kann.

Wird aber ein Trennkörper so eingeführt, dass ein vollständiger Abschluss eines Reaktionsraums auftritt, können Informations- und/oder Reizweiterleitungen innerhalb einer vollständigen in einem Aufnahmebehältnis enthaltenen Zellkultur nicht untersucht werden. Dieser Sachverhalt betrifft insbesondere die Informations- und/oder Reizweiterleitung zwischen von einem Mittel beeinflussten Zellen zu von dem Mittel nicht beeinflussten Zellen zu.

Es ist daher Aufgabe der Erfindung Möglichkeiten vorzuschlagen, mit denen vergleichende Untersuchungen an Zellkulturen, bei gleichen Bedingungen an Zellen, die von einer Testflüssigkeit beeinflusst und Zellen, die von einer Testflüssigkeit nicht beeinflusst sind, vornehmen zu können.

Erfindungsgemäß wird diese Aufgabe mit einem Zellkulturmesssystem, das die Merkmale des Anspruchs 1 aufweist, gelöst. Die Untersuchungen können mit einem erfindungsgemäßen Verfahren unter Verwendung eines solchen Zellkulturmesssystems gemäß Anspruch 9 durchgeführt werden.

Vorteilhafte Ausgestaltungsformen und Weiterbildungen der Erfindung können mit in untergeordneten Ansprüchen bezeichneten Merkmalen erreicht werden.

Ein erfindungsgemäßes Zellkulturmesssystem ist dabei so ausgebildet, dass innerhalb eines Flusskanals, an dem mindestens ein Zufluss und mindestens ein Abfluss für ein flüssiges Kulturmedium vorhanden sind, ein Zellkulturträger mit an diesem kultivierten Zellkulturen angeordnet ist. Durch den Flusskanal wird vom Zufluss zum Abfluss ein flüssiges Kulturmedium hindurchgeführt. Am Flusskanal ist mindestens ein weiterer Zufluss für eine Testflüssigkeit vorhanden. Dieser Zufluss ist in Strömungsrichtung des Kulturmediums zwischen dem Zufluss für das Kulturmedium und dem Abfluss des Flusskanals so angeordnet, dass er das Kulturmedium verdrängt und so die durch den Flusskanal strömende Testflüssigkeit lediglich einen Teil der auf dem Zellkulturträger vorhanden Zellen um- und/oder überströmt. Im Bereich der Zellkultur sind mindestens zwei Sensoren angeordnet, wobei mindestens ein Sensor im Bereich der von Testflüssigkeit unbeeinflussten Zellen und mindestens ein weiterer Sensor im Bereich von mit Testflüssigkeit beeinflussten Zellen angeordnet ist.

Mit diesen Sensoren können dann Zellfunktionen oder Wirkungen des Zellstoffwechsels sowie Veränderungen der Morphologie von Zellen detektiert werden.

Eine Testflüssigkeit gelangt so in einen Flusskanal und bei Einhaltung bestimmter Strömungsbedingungen erfolgt die Verdrängung des durch den Flusskanal hindurchströmenden Kulturmediums durch die Testflüssigkeit so, dass sich zwischen Kulturmedium und Testflüssigkeit eine relative scharfe Grenzfläche beim Durchströmen beider Flüssigkeiten ausbildet und dabei nahezu keine Konvektion und Diffusion von Testflüssigkeit bzw. in einer Testflüssigkeit enthaltenen die Zellen beeinflussenden Mittel außerhalb des Bereichs, der für die Untersuchung des Einflusses einer Testflüssigkeit genutzt werden soll, erfolgt.

Dies kann beispielsweise durch geeignete Steuerung oder Regelung der durch den Flusskanal geführten Volumenströme von Kulturmedium und/oder Testflüssigkeit erreicht werden. Es kann ausreichend sein, Kulturmedium mit einem konstant gehaltenen Volumenstrom über Zu- und Abfluss durch den Flusskanal zu führen und lediglich den Volumenstrom der Testflüssigkeit, der über den weiteren Zufluss in den Flusskanal eingeführt wird, entsprechend gezielt zu beeinflussen.

Eine Testflüssigkeit kann als solche bereits eine zellbeeinflussende Wirkung hervorrufen. In einer Testflüssigkeit können aber auch solche Mittel in gelöster Form enthalten sein oder eine Testflüssigkeit eine Suspension sein.

Um zu erreichen, dass ausschließlich Flüssigkeit durch den Flusskanal hindurchgeführt wird, die keine Gasblasen enthält, kann an einem oder mehreren Zuflüssen eine semipermeable Membran oder ein Mikrosieb, als Beispiele für Blasenfallen, angeordnet sein, über die/das ein flüssiges Kulturmedium und/oder eine Testflüssigkeit in den Flusskanal einströmen kann/können. Für die Beeinflussung der zugeführten Volumenströme von Kulturmedium und/oder Testflüssigkeit können an sich bekannte und geeignete Mikrodosiersysteme eingesetzt werden.

Die bei den vergleichenden Untersuchungen eingesetzten Sensoren, die im Bereich der von Testflüssigkeit unbeeinflussten und im Bereich von Testflüssigkeit beeinflussten Zellen angeordnet sind, sollten jeweils gleiche Sensoren sein, die in gleicher Form sensitiv sind. Es können aber auch mehrere, z.B. Paare oder Tripel von gleichen Sensoren mit unterschiedlicher Sensitivität in den nicht oder in den beeinflussten Bereichen der Zellkultur angeordnet sein. Dadurch können dann gleichzeitig unterschiedliche vergleichende Untersuchungen an Zellen durchgeführt werden.

Insbesondere bei Untersuchungen, bei denen auch eine Informations- und/oder Reizweiterleitung zwischen Zellen vorgenommen werden sollen, ist es vorteilhaft, die Sensoren in jeweils gleichen Abständen voneinander anzuordnen. Dies betrifft dann auch zusätzliche Sensoren, die dann in einem von Testflüssigkeit unbeeinflussten Bereich angeordnet sind und dabei dann mindestens zwei solcher Sensoren unterschiedliche Abstände zu Zellen, die von Testflüssigkeit beeinflusst worden sind, aufweisen.

Wie bereits angesprochen, soll mindestens ein Zufluss von Testflüssigkeit zwischen einem Zufluss für Kulturmedium und dem mindestens einen Abfluss an einem Flusskanal angeordnet sein. Dieser mindestens eine weitere Zufluss für Testflüssigkeit sollte dann in einem Winkel zwischen 0° und 90°, bevorzugt 45° und 90° in Bezug zur Strömungsrichtung des Kulturmediums in den Flusskanal münden, wobei dies zumindest für den Bereich des Flusskanals zutrifft, in dem flüssiges Kulturmedium und Testflüssigkeit dann gemeinsam in Richtung eines Abflusses aus dem Flusskanal strömen.

Bei der Erfindung besteht auch vorteilhaft die Möglichkeit, Sensoren unmittelbar am oder auch im Zellkulturträger auszubilden, was der Möglichkeit eines modularen Aufbaus eines Zellkulturmesssystems förderlich ist.

Ein an einem erfindungsgemäßen Zellkulturmesssystem einsetzbarer Zellkulturträger kann aus einem polymeren Material gebildet sein. Dieses polymere Material kann vor der eigentlichen Nutzung selektiv bereichsweise an seiner Oberfläche funktionalisiert werden, um selektiv eine Adhäsion von Zellen erreichen oder unterdrücken zu können. Eine solche Funktionalisierung kann durch Bestrahlung mit elektromagnetischer Strahlung im Wellenlängenbereich des ultravioletten Lichts unter gleichzeitigem Einfluss eines Reaktivgases erreicht werden.

Ein solcher Zellkulturträger aber auch andere Teile eines erfindungsgemäßen Zellkulturmesssystems, wie z.B. auch ein Deckelelement können aus einem optisch transparenten polymeren Material gebildet sein. Dadurch erschließt sich zusätzlich die Möglichkeit an der Zellkultur mikroskopierende Untersuchungen vornehmen zu können.

Bei dem erfindungsgemäßen Zellkulturmesssystem ist es außerdem günstig, den Flusskanal geometrisch so zu gestalten, dass im Bereich eines Zuflusses für Testflüssigkeit eine größere Breite vorhanden ist, als dies auf den Bereich an dem ein Zufluss für Kulturmedium angeordnet ist, zutrifft. Dadurch wird die Strömungsgeschwindigkeit des flüssigen Kulturmediums im Bereich des Zuflusses für Testflüssigkeit reduziert und in diesem Bereich eine überwiegend laminare Strömung ausgebildet, die deutlich besser mit einer scharfen Grenzschicht die Ausbildung eines Bereichs, in dem Zellen von Testflüssigkeit beeinflusst werden, durch Verdrängung von flüssigem Kulturmedium durch Testflüssigkeit ausgenutzt werden kann. Es sollte eine laminare Strömung von Testflüssigkeit und Kulturmedium eingehalten sein.

Bei einem erfindungsgemäßen Zellkulturmesssystem können, wie bereits angesprochen, unterschiedliche Sensoren eingesetzt werden, wobei dies auf jeweils gleiche Sensoren aber auch Sensoren mit unterschiedlicher Sensitivität, die in den von Testflüssigkeit beeinflussten und von Testflüssigkeit nicht beeinflussten Bereichen angeordnet sein können, zutrifft. Solche Sensoren können pH-sensitiv, sauerstoffsensitiv, glucosesensitiv, optisch (Morphologie, Fluoreszenz), elektrisch (Elektrische Potentiale) und/oder elektrophysiologisch (Konzentrationen) sensitiv sein.

Bei den vergleichenden Untersuchungen von Zellen sollte mit den Sensoren eine Bestimmung zeitaufgelöst durchgeführt werden, um insbesondere die Möglichkeit der Bestimmung eines Stoffaustauschs, einer Informations- und/oder Reizweiterleitung zu ermöglichen. So können Messsignale zu bestimmten Zeitpunkten erfasst werden, wenn beispielsweise der Zufluss für Testflüssigkeit geöffnet worden ist. Dann wird ein Einfluss durch die Testflüssigkeit auf einen Bereich von Zellen ausgeübt. Es kann dann die Zeit erfasst werden, bis eine Wirkung von Testflüssigkeit auf Zellen auftritt und nachfolgend dann diese Wirkung wiederum Einfluss auf Zellen hat, die in einem Bereich angeordnet sind, der von Testflüssigkeit unbeeinflusst bleibt.

Die jeweiligen zeitlich erfassten Messwerte können dann nachfolgend ausgewertet und die jeweilige Wirkung der Testflüssigkeit ermittelt werden.

An einem erfindungsgemäßen Zellkulturmesssystem können aber auch mehrere Zuflüsse in Strömungsrichtung des flüssigen Kulturmediums am Flusskanal nacheinander angeordnet, vorhanden sein. Dadurch lassen sich unterschiedliche Konzentrationen eines die Zellen beeinflussenden Mittels in unterschiedlichen Bereichen des Flusskanals erreichen, so dass eine differenzierte Beeinflussung von Zellen mit Testflüssigkeit und/oder einem die Zellen beeinflussenden Mittel in differenzierter Form erreichen lässt. Es können aber auch allein oder zusätzlich unterschiedliche Testflüssigkeiten, die unterschiedliche Zellen beeinflussende Mittel enthalten in dieser Form zuführen. So können Zellen unter gleichen Bedingungen, bis auf die jeweils unterschiedliche Zusammensetzung von Testflüssigkeiten, untersucht werden.

Der eine oder auch mehrere Zufluss/Zuflüsse kann/können in definierter Form ab- oder auch angeschaltet/geöffnet werden. So kann beispielsweise Testflüssigkeit über einen vorgebbaren Zeitraum zugeführt und danach die Zufuhr von Testflüssigkeit wieder beendet werden. In dieser Form kann beispielsweise untersucht werden, inwiefern die Wirkung des die Zellen beeinflussenden Mittels wieder nachlässt, teilweise oder vollständig irreversibel ist.

Bei der Erfindung können die Bedingungen bei der Zellkultivierung und der Untersuchung des Zellen beeinflussenden Mittels ohne weiteres konstant gehalten werden und dabei vergleichende Untersuchungen an mit einem die Zellen beeinflussenden Mittel und gleichzeitig bei den gleichen Bedingungen auch an Zellen, die von einem solchen Mittel nicht beeinflusst werden, vorgenommen werden. Die unbeeinflussten Zellen stellen eine Referenz für die vergleichenden Untersuchungen dar.

Ein erfindungsgemäßes Zellkulturmesssystem kann einen offenen aber auch einen geschlossenen Flusskanal aufweisen. Durch gezielte Beeinflussung der durch den Flusskanal geführten Volumenströme von flüssigem Kulturmedium und Testflüssigkeit können ganz gezielt der Bereich, der von einem zellenbeeinflussenden Mittel auch die jeweiligen Zellen beeinflusst, in seiner Größe und mit dem entsprechenden Anteil von Zellen beeinflusst werden.

Bei der Durchführung der Untersuchungen kann so vorgegangen werden, dass zuerst Zellen an einem Zellkulturträger kultiviert werden, was auch im erfindungsgemäßen Zellkulturmesssystem und dabei auch bereits im Flusskanal erfolgen kann. Es kann dabei ein Kulturmedium ohne Testflüssigkeit alle Zellen um- und/oder überströmen. Nachfolgend kann dann für eine vorgebbare Zeitdauer mindestens eine Testflüssigkeit mit bekannter Zusammensetzung so zugeführt werden, dass lediglich ein Teil der Zellen mit Testflüssigkeit um- und/oder überströmt wird. Nach Beendigung der Zufuhr von Testflüssigkeit kann dann wieder ausschließlich Kulturmedium zu allen Zellen geführt werden. Dabei kann eine Detektion mit allen Sensoren während der gesamten Zeit erfolgen. Selbstverständlich können Messsignale dabei kontinuierlich oder auch in vorgebbaren Zeitintervallen intermittierend erfasst werden.

Nachfolgend soll die Erfindung beispielhaft näher erläutert werden.

Dabei zeigen
- Fig. 1: in schematischer Form eine Draufsicht auf ein Beispiel eines erfindungsgemäßen Zellkulturmesssystems;
- Fig. 2: in schematischer Form ein weiteres Beispiel in Draufsicht auf ein erfindungsgemäßes Zellkulturmesssystem;
- Fig. 3: in schematischer Form eine Draufsicht auf ein Beispiel eines erfindungsgemäßen Zellkulturmesssystems mit mehreren Zuflüssen für Testflüssigkeit; und
- Fig. 4: ein Beispiel eines erfindungsgemäßen Zellkulturmesssystems in einer Draufsicht mit zwei Abflüssen und einem Zufluss für ein Kulturmedium.

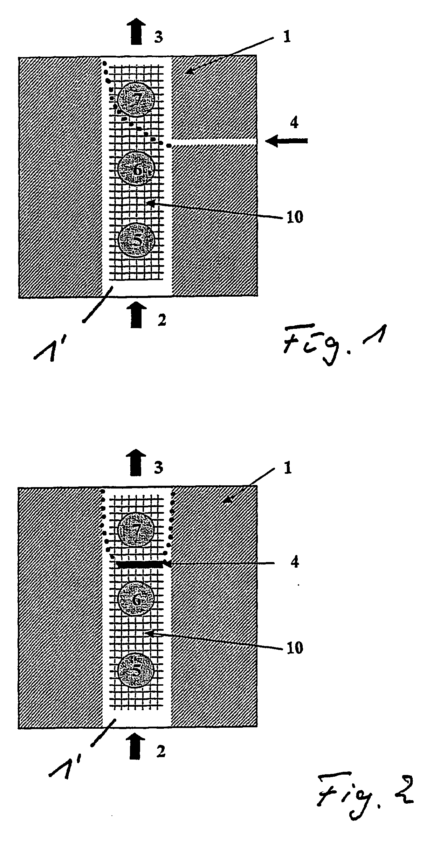

Bei dem in Fig. 1 gezeigten Beispiel eines erfindungsgemäßen Zellkulturmesssystems 1 ist ein Flusskanal 1' mit einem Zufluss 2 für ein flüssiges Kulturmedium vorhanden, von dem ausgehend das flüssige Kulturmedium durch den Flusskanal 1' in Richtung auf einen Abfluss 3 des Flusskanals 1' strömt. Aus Fig. 1 wird deutlich, dass der Flusskanal 1' einen verbreiterten Bereich/Querschnitt zwischen dem Zufluss 2 und dem Abfluss 3 aufweist. Dort ist ein Zellkulturträger 10, auf dem Zellkulturen angeordnet/ausgebildet sind, angeordnet.

Am Zellkulturträger 10 sind Sensoren 5, 6 und 7 vorhanden, mit denen Zellfunktionen detektiert werden können.

Die Sensoren 5, 6 und 7 sind in gleichen Abständen zueinander und bei diesem Beispiel auf einer diagonalen Achse, des hier in quadratischer Form ausgebildeten Zellkulturträgers 10, angeordnet.

An einer Seite des Flusskanals 1' ist ein weiterer Zufluss 4 für eine Testflüssigkeit vorhanden, durch den in definierter Form Testflüssigkeit in den Flusskanal 1' einströmen kann. Der Zufluss 4 ist hier in einem 90°-Winkel in Bezug zur Strömungsrichtung des Kulturmediums zwischen dem Zufluss 2 und dem Abfluss 3 ausgerichtet.

Mit Fig. 1 wird außerdem verdeutlicht, wie die durch den Zufluss 4 einströmende Testflüssigkeit flüssiges Kulturmedium verdrängt und ein Bereich mit kultivierten Zellen auf dem Zellkulturträger 10 von der Testflüssigkeit oder einem in einer Testflüssigkeit enthaltenen, die Zellen beeinflussenden Mittel, beeinflusst wird. Bei diesem Beispiel ist es die rechte obere Ecke in der Darstellung von Fig. 1, in dem der Sensor 7 angeordnet ist, mit dem eine Detektion von mit Testflüssigkeit beeinflussten Zellen möglich ist. Mit den Sensoren 5 und 6, die in Bereichen, die von Testflüssigkeit unbeeinflusst bleiben, angeordnet sind, kann die gleiche Detektion an von Testflüssigkeit unbeeinflussten Zellen durchgeführt werden.

Das in Fig. 2 gezeigte Beispiel unterscheidet sich vom Beispiel nach Fig. 1 lediglich durch die Ausbildung des Zuflusses 4 für Testflüssigkeit. Dieser ist nicht durch die Wandung des Flusskanals 1' seitlich von der Außenseite geführt, sondern der Zufluss 4 für Testflüssigkeit ist bei diesem Beispiel zwar an der gleichen Seite und auch in der Mitte des Zellkulturträgers 10 aber am Zellkulturträger 10 ausgebildet.

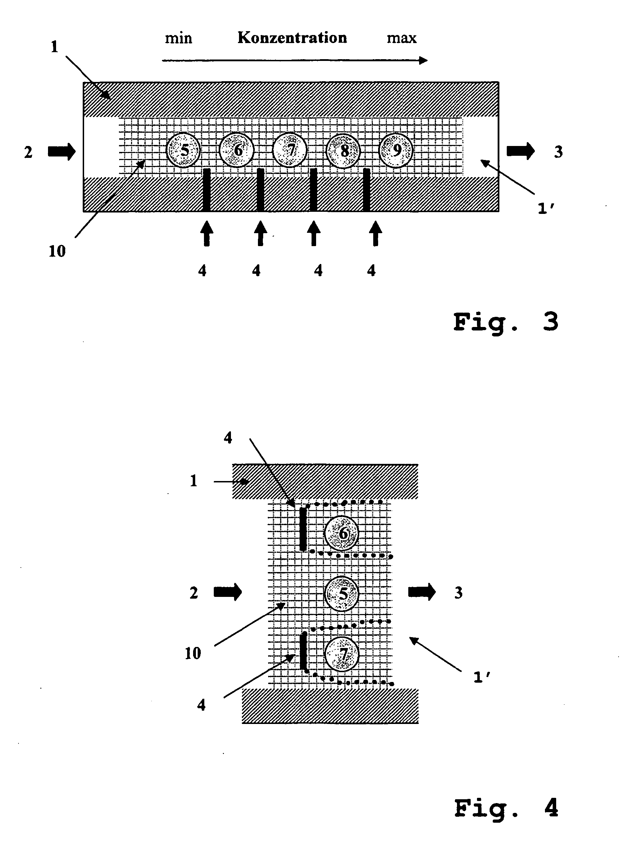

Bei dem in Fig. 3 gezeigten Beispiel sind mehrere Zuflüsse 4 für Testflüssigkeit an einer Seite des Flusskanals 1' vorhanden und hier äquidistant zueinander angeordnet.

Dadurch kann eine gezielte Beeinflussung einer Konzentration an Testflüssigkeit oder einem Mittel, das die Zellen beeinflusst, das in der Testflüssigkeit enthalten ist, lokal differenziert verändert werden, wie dies mit dem oberhalb dargestellten Pfeil verdeutlicht ist. Dementsprechend werden Zellen, die weiter in Richtung des Abflusses 3 nach Zuflüssen 4 für Testflüssigkeit angeordnet sind, mit einer erhöhten Konzentration beaufschlagt. Durch Anordnung mehrerer Sensoren 7 in diesem Bereich kann auch der konzentrationsabhängige Einfluss auf die Zellen detektiert werden.

Bei dem in Fig. 4 gezeigten Beispiel eines erfindungsgemäßen Zellkulturmesssystems ist ein Zufluss 2 in der Mitte an einem Flusskanal 1' vorhanden. Das zugeführte flüssige Kulturmedium strömt dann in Richtung des Abflusses 3.

Es sind außerdem bei diesem Beispiel zwei Zuflüsse 4 für Testflüssigkeit im Flusskanal 1' so angeordnet, dass Zellen und Sensoren 6 und 7 mit Testflüssigkeit um- und/oder überströmt werden, wohingegen ein von Testflüssigkeit unbeeinflusster Bereich von Zellen und der Sensor 5 verbleibt. So können Untersuchungen unter gleichen Bedingungen an Zellen mit mehreren Testflüssigkeiten, also auch mit unterschiedlichen Zellen beeinflussenden Mitteln durchgeführt werden.

Im mittleren Bereich des Flusskanals 1' wird lediglich Kulturmedium hindurchgeführt.

## Patentansprüche

1. Zellkulturmesssystem für vergleichende Untersuchungen an Zellkulturen, bei dem Zellkulturen auf einem Zellkulturträger kultiviert sind, dabei die Zellen innerhalb eines Flusskanals mit einem Zufluss und einem Abfluss für ein flüssiges Kulturmedium und mindestens zwei Sensoren angeordnet sind,
**dadurch gekennzeichnet, dass** mindestens ein weiterer Zufluss (4) für eine Testflüssigkeit in Strömungsrichtung des Kulturmediums zwischen dem Zufluss (2) für das Kulturmedium und dem Abfluss (3) des Flusskanals (1'), so angeordnet ist, dass durch Verdrängung des Kulturmediums die durch den Flusskanal (1') strömende Testflüssigkeit lediglich einen Teil der auf dem Zellkulturträger (10) vorhanden Zellen um- und/oder überströmt und dabei mindestens ein Sensor (5) im Bereich der von Testflüssigkeit unbeeinflussten Zellen und mindestens ein weiterer Sensor (6 bis 9) im Bereich von mit Testflüssigkeit beeinflussten Zellen angeordnet ist.

2. Zellkulturmesssystem nach Anspruch 1, **dadurch gekennzeichnet, dass** der durch den Flusskanal (1') geführte Volumenstrom von Kulturmedium und/oder Testflüssigkeit steuer- oder regelbar ist.

3. Zellkulturmesssystem nach Anspruch 1 oder 2, **dadurch gekennzeichnet, dass** die Testflüssigkeit ein die Zellen beeinflussendes Mittel ist oder ein solches enthält.

4. Zellkulturmesssystem nach einem der vorhergehenden Ansprüche, **dadurch gekennzeichnet, dass** gleiche Sensoren (5 bis 9) im Bereich der von Testflüssigkeit unbeeinflussten Zellen und im Bereich von mit Testflüssigkeit beeinflussten Zellen angeordnet sind.

5. Zellkulturmesssystem nach einem der vorhergehenden Ansprüche, **dadurch gekennzeichnet, dass** mindestens ein Zufluss (4) für Testflüssigkeit in einem Winkel zwischen 0° und 90° in Bezug zur Strömungsrichtung des Kulturmediums in den Flusskanal (1') mündet.

6. Zellkulturmesssystem nach einem der vorhergehenden Ansprüche, **dadurch gekennzeichnet, dass** der Flusskanal (1') im Bereich eines Zuflusses (4) für Testflüssigkeit eine größere Breite aufweist, als im Bereich an dem der Zufluss (2) für Kulturmedium angeordnet ist.

7. Zellkulturmesssystem nach einem der vorhergehenden Ansprüche, **dadurch gekennzeichnet, dass** zumindest ein Zellkulturträger (10) aus einem polymeren Material gebildet und selektiv bereichsweise an seiner Oberfläche funktionalisiert ist.

8. Zellkulturmesssystem nach einem der vorhergehenden Ansprüche, **dadurch gekennzeichnet, dass** pH-sensitive, sauerstoffsensitive, glucosesensitive, optisch, elektrisch und/oder elektrophysiologisch sensitive Sensoren (5 bis 9)vorhanden und/oder unmittelbar am oder im Zellkulturträger (10) ausgebildet sind.

9. Verfahren für vergleichende Untersuchungen an Zellkulturen unter Verwendung eines Zellkulturmesssystems nach einem der Ansprüche 1 bis 8, **dadurch gekennzeichnet, dass** ein Kulturmedium über einen Zufluss (2) zu mindestens einem Abfluss (3) durch einen Flusskanal (1'), in dem Zellen auf einem Zellkulturträger (10) angeordnet sind, hindurchströmt und die Zellen um- und/oder überströmt und
über mindestens einen weiteren Zufluss (4) eine Testflüssigkeit dem Flusskanal (1') zwischen einem Zufluss (2) und einem Abfluss (3) zugeführt wird;
wobei die Strömung der Testflüssigkeit das Kulturmedium bereichsweise verdrängt und die Testflüssigkeit einen Bereich von Zellen beeinflusst und ein Teil der Zellen unbeeinflusst bleibt und
mit mindestens einem Sensor (5) Zellfunktionen von von Testflüssigkeit unbeeinflussten und mit mindestens einem weiteren Sensor (6 bis 9) Zellfunktionen von von Testflüssigkeit beeinflussten Zellen bestimmt werden.

10. Verfahren nach Anspruch 9, **dadurch gekennzeichnet, dass** die Bestimmung zeitaufgelöst durchgeführt wird.

11. Verfahren nach Anspruch 9 oder 10, **dadurch gekennzeichnet, dass** die Reizweiterleitung und/oder ein Stoffaustausch von Zellen aus von Testflüssigkeit beeinflussten Bereichen zu von Testflüssigkeit unbeeinflussten Zellen bestimmt wird/werden.

12. Verfahren nach einem der Ansprüche 9 bis 11, **dadurch gekennzeichnet, dass** Testflüssigkeit mit unterschiedlichen Zellen beeinflussenden Mitteln oder Konzentrationen solcher Zellen beeinflussenden Mitteln über mehrere Zuflüsse (4) dem Flusskanal (1') in Strömungsrichtung des Flusskanals (1) zugeführt und so die Zellen bereichsweise differenziert beeinflusst werden.

13. Verfahren nach einem der Ansprüche 9 bis 12, **dadurch gekennzeichnet, dass** ein polymerer Zellkulturträger (10) eingesetzt wird, dessen Oberfläche bereichsweise und/oder selektiv durch Bestrahlung mit elektromagnetischer Strahlung aus dem Wellenlängenspektrum des ultravioletten Lichts in einer Reaktivgasatmosphäre funktionalisiert worden ist.

14. Verfahren nach einem der Ansprüche 9 bis 13, **dadurch gekennzeichnet, dass** der Volumenstrom des durch den Flusskanal (1') hindurchströmenden Kulturmediums und/oder der Testflüssigkeit gesteuert oder geregelt wird.

15. Verfahren nach einem der Ansprüche 9 bis 14, **dadurch gekennzeichnet, dass** Zellen unter gleichen Bedingungen kultiviert werden,
dabei über eine bestimmte Zeitdauer ein Kulturmedium alle Zellen um- und/oder überströmt, nachfolgend mindestens eine Testflüssigkeit über einen Zufluss (4) und eine vorgebbare Zeitdauer so zugeführt wird, dass ein Teil der Zellen von Testflüssigkeit beeinflusst und ein Teil der Zellen unbeeinflusst bleibt und anschließend, die Zufuhr von Testflüssigkeit beendet und dann alle Zellen wieder ausschließlich von Kulturmedium um- und/oder überströmt werden und wobei über die gesamte Zeit Zellfunktionen bestimmt werden.

## Claims

1. A cell culture measuring system for comparative investigations on cell cultures, in which cell cultures are cultivated on a cell culture support, in which case the cells are arranged within a flow passage with an inlet and an outlet for a liquid culture medium and at least two sensors,
**characterised in that** at least one further inlet (4) for a test liquid is arranged in the direction of flow of the culture medium between the inlet (2) for the culture medium and the outlet (3) of the flow passage (1') such that by displacement of the culture medium the test liquid flowing through the flow passage (1') flows around and/or over merely some of the cells present on the cell culture support (10), and in such case at least one sensor (5) is arranged in the region of the cells which are uninfluenced by test liquid and at least one further sensor (6 to 9) is arranged in the region of cells which are influenced with test liquid.

2. A cell culture measuring system according to Claim 1, **characterised in that** the volume flow of culture medium and/or test liquid which is conducted through the flow passage (1') can be controlled or regulated.

3. A cell culture measuring system according to Claim 1 or 2, **characterised in that** the test liquid is a medium which influences the cells, or contains such.

4. A cell culture measuring system according to one of the preceding claims, **characterised in that** identical sensors (5 to 9) are arranged in the region of the cells which are uninfluenced by test liquid and in the region of cells which are influenced with test liquid.

5. A cell culture measuring system according to one of the preceding claims, **characterised in that** at least one inlet (4) for test liquid opens into the flow passage (1') at an angle of between 0° and 90° with respect to the direction of flow of the culture medium.

6. A cell culture measuring system according to one of the preceding claims, **characterised in that** the flow passage (1') in the region of an inlet (4) for test liquid has a greater width than in the region at which the inlet (2) for culture medium is arranged.

7. A cell culture measuring system according to one of the preceding claims, **characterised in that** at least one cell culture support (10) is formed from a polymeric material and is selectively functionalised in regions on its surface.

8. A cell culture measuring system according to one of the preceding claims, **characterised in that** pH-sensitive, oxygen-sensitive, glucose-sensitive, optically, electrically and/or electrophysiologically sensitive sensors (5 to 9) are present and/or are formed directly on or in the cell culture support (10).

9. A method for comparative investigations on cell cultures using a cell culture measuring system according to one of Claims 1 to 8,
**characterised in that** a culture medium flows via an inlet (2) to at least one outlet (3) through a flow passage (1') in which cells are arranged on a cell culture support (10), and flows around and/or over the cells, and
a test liquid is supplied via at least one further inlet (4) to the flow passage (1') between an inlet (2) and an outlet (3);
the flow of the test liquid displacing the culture medium in regions and the test liquid influencing a region of cells and some of the cells remaining uninfluenced, and
cell functions of cells which are uninfluenced by test liquid are determined with at least one sensor (5) and cell functions of cells influenced by test liquid are determined with at least one further sensor (6 to 9).

10. A method according to Claim 9, **characterised in that** the determination is carried out in time-resolved manner.

11. A method according to Claim 9 or 10, **characterised in that** the stimulus transmission and/or an exchange of substances from cells from regions influenced by test liquid to cells which are uninfluenced by test liquid is/are determined.

12. A method according to one of Claims 9 to 11, **characterised in that** test liquid with different agents influencing cells or concentrations of such agents influencing cells are supplied via a plurality of inlets (4) to the flow passage (1') in the direction of flow of the flow passage (1), and thus the cells are influenced in regions in differentiated manner.

13. A method according to one of Claims 9 to 12, **characterised in that** a polymeric cell culture support (10) is used, the surface of which has been functionalised in regions and/or selectively by irradiation with electromagnetic radiation from the wavelength spectrum of ultraviolet light in a reactive gas atmosphere.

14. A method according to one of Claims 9 to 13, **characterised in that** the volume flow of the culture medium flowing through the flow passage (1') and/or of the test liquid is controlled or regulated.

15. A method according to one of Claims 9 to 14, **characterised in that** cells are cultivated under identical conditions,
in so doing, a culture medium flows around and/or over all the cells over a certain time period,
subsequently at least one test liquid is supplied via an inlet (4) and over a presettable time period such that some of the cells are influenced by test liquid and some of the cells remain uninfluenced, and then the supply of test liquid is ended and then all the cells are flowed around and/or over again exclusively by culture medium, and with cell functions being determined over the entire time.

## Revendications

1. Système de mesure de cultures cellulaires pour des essais comparatifs sur des cultures cellulaires, dans lequel des cultures cellulaires sont cultivées sur un support de cultures cellulaires, les cellules étant agencées à l'intérieur d'un canal d'écoulement avec un orifice d'arrivée et un orifice d'évacuation pour un milieu de culture liquide et au moins deux capteurs, **caractérisé en ce qu'**au moins un orifice d'arrivée supplémentaire (4) pour un liquide d'essai est agencé dans la direction d'écoulement du milieu de culture et entre l'orifice d'arrivée (2) pour le milieu de culture et l'orifice d'évacuation (3) du canal d'écoulement (1') de telle sorte que, sous l'effet d'un déplacement du milieu de culture, le liquide d'essai s'écoulant par le canal d'écoulement (1') contourne et/ou submerge uniquement une partie des cellules présentes sur le support de cultures cellulaires (10), au moins un capteur (5) étant alors agencé dans la zone des cellules non affectées par le liquide d'essai, et au moins un capteur supplémentaire (6 à 9) étant agencé dans la zone des cellules affectées par le liquide d'essai.

2. Système de mesure de cultures cellulaires selon la revendication 1, **caractérisé en ce que** le débit volumique passant par le canal d'écoulement (1') peut être piloté ou régulé par le milieu de culture et/ou le liquide d'essai.

3. Système de mesure de cultures cellulaires selon la revendication 1 ou 2, **caractérisé en ce que** le liquide d'essai est un agent affectant les cellules, ou contient un tel agent.

4. Système de mesure de cultures cellulaires selon l'une des revendications précédentes, **caractérisé en ce que** des capteurs identiques (5 à 9) sont agencés dans la zone des cellules non affectées par le liquide d'essai et dans la zone des cellules affectées par le liquide d'essai.

5. Système de mesure de cultures cellulaires selon l'une des revendications précédentes, **caractérisé en ce qu'**au moins un orifice d'arrivée (4) pour le liquide d'essai débouche dans le canal d'écoulement (1') selon un angle compris entre 0 et 90° par rapport à la direction d'écoulement du milieu de culture.

6. Système de mesure de cultures cellulaires selon l'une des revendications précédentes, **caractérisé en ce que** le canal d'écoulement (1') présente, dans la zone d'un orifice d'arrivée (4) pour le liquide d'essai, une largeur plus grande que dans la zone dans laquelle est agencé l'orifice d'arrivée (2) pour le milieu de culture.

7. Système de mesure de cultures cellulaires selon l'une des revendications précédentes, **caractérisé en ce qu'**au moins un support de cultures cellulaires (10) est formé dans un matériau polymère et est fonctionnalisé, sélectivement par zones, sur sa surface.

8. Système de mesure de cultures cellulaires selon l'une des revendications précédentes, **caractérisé en ce que** des capteurs (5 à 9) sensibles au pH, sensibles à l'oxygène, sensibles au glucose, présentant une sensibilité optique, électrique et/ou électrophysiologique, sont présents et/ou configurés immédiatement sur ou dans le support de cultures cellulaires (10).

9. Procédé pour des essais comparatifs portant sur des cultures cellulaires par utilisation d'un système de mesure de cultures cellulaires selon l'une des revendications 1 à 8, **caractérisé en ce qu'**un milieu de culture passe par un orifice d'arrivée (2) pour arriver à au moins un orifice d'évacuation (3) en passant par un canal d'écoulement (1') dans lequel sont agencées des cellules sur un support de cultures cellulaires (10), et contourne et/ou submerge les cellules, et un liquide d'essai est envoyé au canal d'écoulement (1') entre un orifice d'arrivée (2) et un orifice d'évacuation (3) par au moins un orifice d'arrivée supplémentaire (4) ;
dans lequel l'écoulement du liquide d'essai déplace par zones le milieu de culture, et le liquide d'essai affecte une zone de cellules et une partie des cellules reste non affectée, et
des fonctions cellulaires de cellules non affectées par le liquide d'essai sont déterminées à l'aide d'au moins un capteur (5), et des fonctions cellulaires de cellules affectées par le liquide d'essai sont déterminées à l'aide d'au moins un capteur supplémentaire (6 à 9).

10. Procédé selon la revendication 9, **caractérisé en ce que** la détermination a lieu avec une résolution temporelle.

11. Procédé selon la revendication 9 ou 10, **caractérisé en ce que** la transmission de l'excitation et/ou un échange de matière de cellules est/sont déterminé(es) à partir des zones affectées par le liquide d'essai jusqu'aux cellules non affectées par le liquide d'essai.

12. Procédé selon l'une des revendications 9 à 11, **caractérisé en ce que** le liquide d'essai avec des agents affectant différentes cellules, ou des agents affectant la concentration de telles cellules, est envoyé par plusieurs orifices d'arrivée (4) au canal d'écoulement (1') dans la direction d'écoulement du canal d'écoulement (1), les cellules étant ainsi affectées d'une manière différenciée par zones.

13. Procédé selon l'une des revendications 9 à 12, **caractérisé en ce qu'**on utilise un support de cultures cellulaires polymère (10), dont la surface a été fonctionnalisée par zones et/ou sélectivement par irradiation d'un rayonnement électromagnétique appartenant au spectre de longueurs d'onde de la lumière ultraviolette, dans une atmosphère d'un gaz réactif.

14. Procédé selon l'une des revendications 9 à 13, **caractérisé en ce que** le débit volumique du milieu de culture passant par le canal d'écoulement (1') et/ou du liquide d'essai est piloté ou régulé.

15. Procédé selon l'une des revendications 9 à 14, **caractérisé en ce que** les cellules sont cultivées dans des conditions identiques, où pendant un certain laps de temps, un milieu de culture contourne et/ou submerge toutes les cellules, puis au moins un liquide d'essai est, par un orifice d'arrivée (4) et sur un laps de temps prédéfinissable, amené de telle sorte qu'une partie des cellules soit affectée par le liquide d'essai et qu'une partie des cellules demeure non affectée, puis on met fin à l'arrivée du liquide d'essai, et ensuite toutes les cellules sont de nouveau, à l'exclusion du milieu de culture, contournées et/ou submergées, des fonctions cellulaires étant déterminées sur la totalité du temps.
